# EUROPÄISCHE PATENTANMELDUNG

(11) **EP 1 103 259 A1**
(43) Veröffentlichungstag der Anmeldung: **30.05.2001**
(21) Anmeldenummer: 00124140.5
(22) Anmeldetag: 07.11.2000
(51) Int. Cl.: A61K 31/205, A23L 1/30

(54) **Verwendung von y-Butyrobetain Salzen zur Herstellung von Zubereitungen für die menschliche und tierische Ernährung**

(30) Priorität: 25.11.1999 DE 19956772
(71) Anmelder: BASF AKTIENGESELLSCHAFT, 67056 Ludwigshafen (DE)
(72) Erfinder: Eichler, Knut, Dr., SG/Singapore 259333 (ML); Jaedicke, Hagen, Dr., 67069 Ludwigshafen (DE); Krämer, Klaus, Dr., 76829 Landau (DE); Hasselwander, Oliver, Dr., 76829 Landau (DE)

(57) **Zusammenfassung**

Die Erfindung betrifft die Verwendung von γ-Butyrobetain Salze der allgemeinen Formel I, 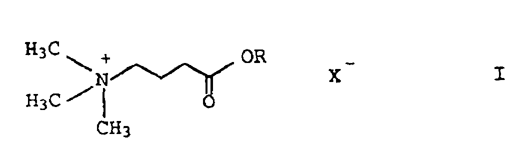 in der unabhängig voneinander R für Wasserstoff oder ein Metallkation steht und X⁻ ein Anion einer Mineralsäure oder organischen Carbonsäure bedeutet, zur Herstellung von Zubereitungen für die menschliche und tierische Ernährung.

## Beschreibung

Die Erfindung betrifft die Verwendung von γ-Butyrobetain Salzen zur Herstellung von Zubereitungen für die menschliche und tierische Ernährung.

γ-Butyrobetain stellt in der Biosynthese von L-Carnitin dessen direkte physiologische Vorstufe dar. Im letzten Schritt der biochemischen Synthese erfolgt die Transformation von γ-Butyrobetain zu L-Carnitin durch Vitamin C abhängige Hydroxylierung mit Hilfe des Enzyms γ-Butyrobetainhydroxylase.

Anhand von Fütterungsversuchen an Ratten [A. Sandor, Biochimica et Biophysica Acta, 1083 (1991) 135-138] konnte gezeigt werden, daß die Aufnahme von γ-Butyrobetain zu einem Anstieg des L-Carnitin-Levels in zahlreichen Organen wie z.B. in der Leber, in den Nieren, im Gehirn und im Muskelgewebe führt.

In der deutschen Patentschrift DE-C-32 00 016 wird die Verwendung von γ-Butyrobetain als pharmazeutischen Wirkstoff bei der Behandlung von L-Carnitinmangelsyndromen beschrieben.

EP-A-0 781 554 beschreibt die Verwendung von γ-Butyrobetainhaltigen pharmazeutischen Zubereitungen zur Behandlung von durch Hydroxylradikale verursachte Erkrankungen.

Es war die Aufgabe der vorliegenden Erfindung, stabile Verbindungen als L-Carnitinersatz für die Verwendung im Lebensmittel- und Tierernährungsbereich bereitzustellen.

Diese Aufgabe wurde gelöst durch die Verwendung von γ-Butyrobetain Salze der allgemeinen Formel I, 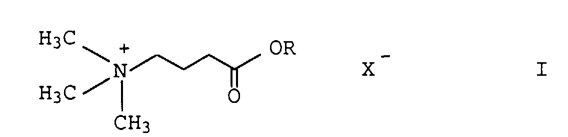 in der unabhängig voneinander R für Wasserstoff oder ein Metallkation steht und X⁻ ein Anion einer Mineralsäure oder organischen Carbonsäure bedeutet, zur Herstellung von Zubereitungen für die menschliche und tierische Ernährung.

Als Metallkation für R sind Alkali- oder Erdalkalimetallkationen, beispielsweise Natrium, Kalium, Lithium, Calcium oder Magnesium sowie diverse für den Organismus essentielle Schwermetallionen wie Zink, Mangan, Eisen oder Kupfer zu verstehen.

Bevorzugte Reste für R sind Wasserstoff, Natrium, Calcium, Magnesium oder Zink. Besonders bevorzugt steht R für Wasserstoff.

Als Beispiele für die in Formel I für X stehenden Anionen mineralischen Ursprungs seien u.a. Chlorid, Bromid, Sulfat, Nitrat, Phosphat, Metaphosphat und Perchlorat genannt. Bevorzugtes Anion aus der Gruppe der Mineralsäuren ist das Chloridion.

Unter den Begriff organische Carbonsäuren sind ohne Einschränkung alle physiologisch unbedenklichen organischen Carbonsäure, sowohl Mono-, Di- und Tricarbonsäuren als auch deren Salze zu verstehen, die mit γ-Butyrobetain stabile, nicht-hygroskopische Salze bilden. Geeignete organische Carbonsäuren sind zum Beispiel in Fortschritte der Arzneimittelforschug, 1966, Birkhäuser Verlag, Bd. 10, S. 224-285 aufgelistet.

Bevorzugt sind solche organische Carbonsäuren oder deren Salze zu nennen, die bei Raumtemperatur amorph oder kristallin vorliegen.

Dazu gehören folgende Anionen: Acetat, Adipat, Alginat, Ascorbat, Aspartat, Benzoat, Benzolsulfonat, Butyrat, Camphersulfonat, Citrat, Digluconat, Dodecylsulfat, Fumarat, Gluconat, Laktat, Lipoat, Maleat, Methansulfonat, Nikotinat, Orotat, Oxalat, Succinat und Tartrat.

Ferner zu nennen sind Anionen mittel- und langkettiger Fettsäuren, z.B. Caprinsäure, Laurinsäure, Palmitinsäure, Staearinsäure oder Arachinsäure, hier bevorzugt Stearinsäure.

Besonders bevorzugt werden im Rahmen der Erfindung folgende Anionen organischer Carbonsäuren verwendet: Ascorbat, Aspartat, Citrat, Fumarat, Gluconat, Laktat, Lipoat, Nikotinat, Orotat und Tartrat, ganz besonders bevorzugt Tartrat und Ascorbat.

Bei Verwendung von Di- oder Tri-Carbonsäuren kommen Salze im Molverhältnis 1 : 1 bis 1 : 3 (Säure : γ-Butyrobetain) in Frage.

Die erfindungsgemäße Verwendung der γ-Butyrobetain Salze der allgemeinen Formel I zeichnet sich dadurch aus, daß die Salze nur eine sehr geringe Hygroskopizität und - im Gegensatz zu salzfreiem γ-Butyrobetain - eine erhöhte Bioverfügbarkeit aufweisen.

Die Herstellung der erfindungsgemäßen γ-Butyrobetain Salze der Formel I kann beispielsweise durch Mischen von γ-Butyrobetain und einer der o.g. Mineralsäuren oder organischen Carbonsäuren im Mol-Verhältnis 1:0,3 bis 1:2, bevorzugt 1:0,5 bis 1:1,5 (γ-Butyrobetain : Säure) in einem polaren organischen Lösungsmittel, beispielsweise einem Alkohol wie Methanol, Ethanol oder Isopropanol oder einem Keton wie Aceton oder Diethylketon und anschließendes Lösen dieser Mischung bei erhöhten Temperaturen, im allgemeinen bei der Siedetemperatur des eingesetzten Lösungsmittels erfolgen. Das gebildete Salz kann dann in an sich bekannter Weise bei niedrigeren Temperaturen, beispielsweise bei Temperaturen zwischen -20 und +40°C, bevorzugt zwischen 0 und 30°C auskristallisiert und anschließend isoliert werden.

Gegenstand der Erfindung ist insbesondere die Verwendung von γ-Butyrobetain Salzen der allgemeinen Formel I als Lebensmittelzusatzstoff sowie die Verwendung zur Herstellung von Zubereitungen zur Nahrungsergänzung, insbesondere im Bereich der Sportlerernährung.

In der Sportlerernährung können solche Zubereitungen eingesetzt werden, da sie zur Versorgung der Muskeln mit Energie beitragen und somit die Ausdauerleistung fördern und eine verzögerte Ermüdung sowie kürzere Erholungszeiten bewirken können.

Die Verwendung γ-Butyrobetain-Salz-haltiger Präparate beschränkt sich jedoch nicht auf die Sportlerernährung, sondern sie können beispielsweise auch für geriatrische Zwecke, in der Säuglingsnahrung in dietätischen Lebensmitteln sowie generell als sogenannte Nutraceuticals verwendet werden.

Die Darreichungsform als Nutraceutical erfolgt vorzugsweise in Tabletten- oder Kapselform sowie als Pulver oder Granulat. Die Herstellung der Darreichungsformen erfolgt dabei nach Methoden der pharmazeutischen Technologie.

Gegenstand der Erfindung sind darüberhinaus auch Lebensmittel sowie Zubereitungen zur Nahrungsergänzung, enthaltend mindestens ein γ-Butyrobetain Salz der allgemeinen Formel I.

Als Lebensmittel seien hier u.a. mit γ-Butyrobetain Salz angereicherte Milchprodukte wie Quark, Joghurt, Mixmilchgetränke, Milcheisprodukte, Getränke wie Vitamin- oder Energy-Drinks, Getreideprodukte wie Müsli, Müsliriegel oder Backwaren gemeint. Bevorzugt sind in diesem Zusammenhang Lebensmittel für den Sportler und Vegetarier zu nennen.

Die Darreichungsformen für die Zubereitungen zu Nahrungsergänzung umfassen insbesondere alle Arten von Tabletten, sowohl solche, die unzerkaut geschluckt werden, als auch Kau- und Lutschtabletten, sowie solche, die vor der Einnahme in einer Flüssigkeit aufgelöst werden.

Dazu zählen einerseits nicht überzogene Tabletten, sowohl in einschichtiger als auch in mehrschichtiger oder ummantelter Form sowie als Brausetabletten, andererseits überzogene Tabletten wie Filmtabletten oder Dragees. Weitere bevorzugte Darreichungsformen sind Kapseln aus Hart- oder Weichgelatine. Hiervon sind Hartgelatinekapseln in Form von Steckkapseln besonders bevorzugt. Weiterhin können γ-Butyrobetain Salze vorteilhaft als Pulver, beispielsweise mit gasentwickelnden Zusätzen als Brausepulver, oder als Granulat verwendet werden.

Die Wirkstoffmenge pro Dosierungseinheit liegt zweckmäßigerweise zwischen 50 mg und 1000 mg, vorzugsweise zwischen 100 mg und 500 mg (berechnet als γ-Butyrobetain).

Die Zubereitungen zur Nahrungsergänzung können neben γ-Butyrobetain Salz der allgemeinen Formel I die üblichen für die Herstellung dieser Zubereitungen notwendigen Hilfsstoffe enthalten.

Hilfsstoffe sind beispielsweise Füllstoffe, Bindemittel, Gleit-und Formtrennmittel, Fließreguliermittel und Sprengmittel für die Tablettenherstellung, sowie Farb- und Aromastoffe.

Als für die Tablettenherstellung erforderliche Hilfsstoffe können eingesetzt werden:
Füllstoffe, z.B. Lactose, Saccharose, Fructose, Mannit, andere Zucker, Zuckeralkohole und Stärke;
Bindemittel, z.B. Stärke , Gelatine, Celluloseether, Cellulose, Carboxymethylcellulose, Polyvinylpyrrolidon und andere wasserlösliche Polymere;
Sprengmittel, z.B. Stärke, Stärkeether, Alginate, Celluloseester und Pektinderivate;
Gleit- und Formtrennmittel, z.B. Talkum, Stearate, hochdisperses Siliciumdioxid und Silikone.

Pulver zur Verwendung in Kapseln oder zur direkten oralen Verabreichung bestehen aus γ-Butyrobetain Salzen und Fließregulierungsmitteln, wie z.B. hochdispersem Siliciumdioxid, Talkum und Stearaten.

Granulate zur Verwendung in Kapseln oder zur direkten oralen Verabreichung bestehen aus γ-Butyrobetain Salzen und Bindemitteln.

Ferner können zusätzliche Antioxidationsmittel wie butyliertes Hydroxyanisol sowie butyliertes Hydroxytoluol, geschmacksverbessernde Zusatzstoffe, Stabilisierungs-, Emulgier- und Gleitmittel enthalten sein.

Darüberhinaus kann es von Vorteil sein, die erfindungsgemäßen γ-Butyrobetain Salze in Kombination mit anderen Wirkstoffen zu verabreichen.

Dabei kann es sich um folgende Wirkstoffe handeln:

Vitaminen beispielsweise Ascorbinsäure, Tocopherol, Tocopherol-acetat, Vitamin A und Vitamin A-Derivate, Vitamin K und Vitamin K-Derivate oder Vitamin D und Vitamin D-Derivate, Riboflavin, Vitamin B₁₂, Nicotinsäure, Nicotinsäureamid, Pyridoxin Hydrochlorid, Biotin, Folsäure, Folsäurederivate wie Tetrahydrofolsäure, 5-Methyl-tetrahydrofolsäure, 10-Formyl-tetrahydrofolsäure oder 5-Formyl-tetrahydrofolsäure.

Carotinoide wie z.B. β-Carotin, Lycopin, Lutein oder Zeaxanthin.

Mehrfach ungesättigte Fettsäuren, wie z.B. Linolsäure, Linolensäure, Arachidonsäure, Docohexaensäure oder Eicosapentaensäure.

Verbindungen mit Vitamin- oder Coenzymcharakter, z.B. Liponsäure, Carnitin, Cholinchlorid, Taurin, Kreatin, Ubichinone, S-Methyl-methionin oder S-Adenosylmethionin.

Die neben dem Wirkstoff in der Zubereitung enthaltenen Stoffe sowie die bei der Herstellung der Zubereitungen verwendeten Stoffe sind toxikologisch unbedenklich und mit dem jeweiligen Wirkstoff verträglich.

Anhand der folgenden Beispiele soll der Gegenstand der vorliegenden Erfindung näher erläutert werden.

### Beispiel 1

### Herstellung von Bis-γ-butyrobetain-tartrat

19,7 g (0,1 mol) γ-Butyrobetain (mit ca. 24% Wasser) wurden mit 7,7 g (0,05 mol) L-(+)-Weinsäure in 100 ml Isopropanol ca. 30 Minuten unter Rückfluß gekocht. Nach Erhalt einer klaren Lösung wurde das Reaktionsgemisch auf 20°C abgekühlt und das ausgefallene Kristallisat abfiltriert. Man erhielt 16,7 g Bis-y-butyrobetain-bi-tartrat als weißen Feststoff mit einem Schmelzpunkt von 131-137°C.

### Beispiel 2

### Herstellung von γ-Butyrobetain-mono-tartrat

19,7 g (0,1 mol) γ-Butyrobetain (mit ca. 24% Wasser) wurden mit 15 g (0,1 mol) L-(+)-Weinsäure in 100 ml Isopropanol ca. 30 Minuten unter Rückfluß gekocht. Nach Erhalt einer klaren Lösung wurde das Reaktionsgemisch auf 20°C abgekühlt und das ausgefallene Kristallisat abfiltriert. Nach Trocknung erhielt man 28,4 g γ-Butyrobetain-mono-tartrat als weißen Feststoff mit einem Schmelzpunkt von 143-146°C.

### Beispiel 3

### Herstellung von γ-Butyrobetain-ascorbat

19,7 g (0,1 mol) γ-Butyrobetain (mit ca. 24% Wasser) wurden mit 17,6 g (0,1 mol) L-Ascorbinsäure in 150 ml Methanol ca. 5 Minuten auf 60°C erhitzt. Nach Erhalt einer klaren Lösung wurde das Reaktionsgemisch auf 20°C abgekühlt und die Lösung bei vermindertem Druck eingeengt. Das ausgefallene Kristallisat wurde anschließend abfiltriert. Man erhielt 31 g γ-Butyrobetain-ascorbat als weißen Feststoff mit einem Schmelzpunkt von 122-130°C (Zersetzung).

### Beispiel 4

### Kapseln zur Verwendung als Nahrungsergänzung

Es wurden Hart-Gelatine Kapseln mit γ-Butyrobetain-mono-tartrat aus Beispiel 2 entsprechend der nachfolgenden Zusammensetzung hergestellt:

| | |
|---|---|
| γ-Butyrobetain-mono-tartrat | 400 mg |
| Magnesiumstearat | 5 mg |

Magnesiumstearat wurde mit einem Sieb von 0,5 mm Maschenweite gesiebt, γ-Butyrobetain-mono-tartrat wurde zugesetzt, und die beiden Komponenten wurden 20 Minuten intensiv vermischt.

Danach wurde in CONI-SNAP® -Kapseln der Größe 1 abgefüllt.

### Beispiel 5

### γ-Butyrobetain-tartrat haltige Brausetablette

| Formulierung: | |
|---|---|
| γ-Butyrobetain-mono-tartrat (Bsp. 2) | 400 g |
| Ludipress® LCE (Fa. BASF AG) | 680 g |
| Natriumhydrogencarbonat | 600 g |
| Weinsäure | 450 g |
| Aspartam | 30 g |
| Polyethylenglycol 6000 (Fa. Hüls) | 90 g |
| Orangenaroma (Fa. Dragoco) | q.s. |

Alle Komponenten wurden gemischt, durch ein Sieb mit 0,8 mm Maschenweite gegeben und mit hoher Preßkraft bei einer maximalen relativen Luftfeuchtigkeit von 30 % verpreßt.

## Patentansprüche

1. Verwendung von γ-Butyrobetain Salzen der allgemeinen Formel I, 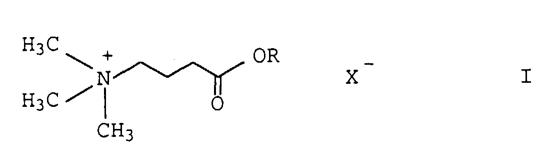 in der unabhängig voneinander R für Wasserstoff oder ein Metallkation steht und X⁻ ein Anion einer Mineralsäure oder organischen Carbonsäure bedeutet, zur Herstellung von Zubereitungen für die menschliche und tierische Ernährung.

2. Verwendung nach Anspruch 1, in der R Wasserstoff und X⁻ ein Anion einer bei Raumtemperatur amorph oder kristallin vorliegenden, organischen Carbonsäure bedeuten.

3. Verwendung nach Anspruch 1, in der R für Wasserstoff und X⁻ für Chlorid stehen.

4. Verwendung nach einem der Ansprüche 1 oder 2, in der X⁻ ein Anion einer organischen Carbonsäure, ausgewählt aus der Gruppe, bestehend aus Weinsäure, Milchsäure, Orotsäure, Asparaginsäure, Zitronensäure, Nikotinsäure, Ascorbinsäure, Liponsäure, Glycolsäure und Fumarsäure bedeutet.

5. Verwendung nach einem der Ansprüche 1 bis 4 zur Herstellung von Zubereitungen zur Nahrungsergänzung.

6. Verwendung nach Anspruch 5 zur Herstellung von Tabletten, Kapseln, Pulvern oder Granulaten.

7. Verwendung nach einem der Ansprüche 1 bis 4 als Lebensmittelzusatzstoff.

8. Zubereitungen zur Nahrungsergänzung, enthaltend mindestens ein γ-Butyrobetain Salz der allgemeinen Formel I, definiert gemäß Anspruch 1.

9. Zubereitungen zur Nahrungsergänzung nach Anspruch 8, in Form von Tabletten, Kapseln, Pulvern oder Granulaten.

10. Lebensmittel, enthaltend mindestens ein γ-Butyrobetain Salz der allgemeinen Formel I, definiert gemäß Anspruch 1, als Lebensmittelzusatzstoff.

11. Lebensmittel nach Anspruch 10 in Form von Getränken, Getreideprodukten, Milchprodukten.
